# DEMANDE DE BREVET EUROPEEN

(11) **EP 2 002 802 A1**
(43) Date de publication de la demande: **17.12.2008**
(21) Numéro de dépôt: 08305236.5
(22) Date de dépôt: 06.06.2008
(51) Int. Cl.: A61F 2/00

(54) **Dispositif de mise en tension d'un implant en matériau souple**

(30) Priorité: 15.06.2007 FR 0755778
(71) Demandeur: Microval, 43240 Saint Just Malmont (FR)
(72) Inventeur: CUILLERON, Olivier, 42270, SAINT PRIEST EN JAREZ (FR); PAIN, Bernard, 43120, MONISTROL SUR LOIRE (FR)
(74) Mandataire: Thivillier, Patrick

(57) **Abrégé**

Le dispositif est assujetti à un ou des fil(s) de traction (F1) et (F2) et destiné à coopérer avec un organe anatomique interne. Il comprend, logé dans un carter de protection étanche et implantable (1), un moyen (4) d'entraînement en rotation d'un disque (2) où sont accouplés le ou le(s) fils de traction (F1) et (F2), ledit moyen d'entraînement (4) étant commandé par injection d'un fluide à partir d'une chambre (7) que présente ledit carter (1).

## Description

L'invention concerne un dispositif de mise en tension, notamment pour implant d'incontinence.

L'invention trouve une application avantageuse dans le traitement de l'incontinence urinaire par insuffisance sphinctérienne, de l'incontinence masculine, et plus généralement lorsqu'un implant de soutien d'un organe anatomique interne doit être mis en tension avec différentes possibilités de réglage.

On connaît, par exemple, l'utilisation d'un implant sous forme d'une bandelette réglable en tension pour le traitement chirurgical de l'incontinence de stress chez les hommes et les femmes. Le système utilisé, connu sous la marque « REMEEX », comprend, pour l'essentiel, sous urétrale, un variateur relié à un régulateur par deux fils de traction. Le système de mise en tension est posé en sous-cutané au niveau par exemple du fascia des muscles abdominaux. La mise en tension des fils s'effectue par l'intermédiaire d'un tournevis afin d'agir sur le variateur.
Cette solution nécessite une incision pour l'introduction de l'extrémité de l'outil.

Toujours dans le traitement de l'incontinence urinaire, une solution ressort de l'enseignement du brevet US 6.117.067 qui décrit un système hydraulique de tension pour implant d'incontinence urinaire. Dans ce système, non invasif, la tension des fils de traction est basée sur la déformation d'un ballon en silicone par injection d'un liquide au travers d'une chambre implantable. Sous l'effet de l'injection du liquide, le gonflement du ballon tire sur les fils reliés à l'implant permettant ainsi d'exercer un effort de traction correspondant, de manière concomitante, au resserrement de l'organe anatomique considéré. Il est également possible de détendre l'implant par aspiration du liquide correspondant à un relâchement des fils.
Cette solution n'est toutefois pas totalement satisfaisante.

Comme indiqué, pour exercer un effort de traction au niveau des extrémités de l'implant, il est nécessaire de gonfler le ballon provoquant une déformation disgracieuse de l'épiderme visible de l'extérieur.
On observe également que la tension exercée à chaque extrémité de l'implant, n'est pas toujours symétrique résultant de la difficulté d'avoir des parois de ballon parfaitement identiques sur toute la surface. En outre, la capacité de réglage n'est pas toujours suffisante.

L'invention s'est fixée pour but de remédier à ces inconvénients d'une manière simple, sûre, efficace et rationnelle.

Le problème que se propose de résoudre l'invention est d'assurer la mise en tension de l'implant sans incision et sans déformation de l'épiderme, avec une plage de réglage importante.

Pour résoudre un tel problème, il a été conçu et mis au point un dispositif de mise en tension d'un implant en matériau souple assujetti à au moins un fil de traction et destiné à coopérer avec un organe anatomique interne. Le dispositif comprend, logé dans un carter de protection étanche et implantable, un moyen d'entraînement en rotation d'un disque où sont accouplés le ou le(s) fils de traction, ledit moyen d'entraînement étant commandé par injection d'un fluide à partir d'une chambre (implantable) que présente ledit carter.

Pour résoudre le problème posé d'obtenir un effort de traction d'intensité égale au niveau de chacune des extrémités de l'implant, le moyen d'entraînement est un piston monté avec capacité de déplacement guidé et étanche dans un logement du carter dans lequel est injecté le fluide.

Pour résoudre le problème posé de l'entraînement en rotation du disque sous l'effet du déplacement du piston, le disque d'accouplement du ou des fil(s) présente une partie débordant dans l'alésage du carter et apte à être sollicité sus un effet de déplacement du piston pour provoquer, d'une manière concomitante, l'entraînement en rotation dudit disque.

Avantageusement, le piston a une forme semi-circulaire, d'une manière complémentaire à celle de son logement.

Selon une autre caractéristique, la chambre implantable est fixée dans un support coaxial au carter et en communication par au moins un orifice avec le logement du piston.

Pour résoudre le problème posé d'assurer une mise en tension symétrique de l'implant, les fils de traction de l'implant, sont accouplés à deux orifices diamétralement opposés formés dans l'épaisseur du disque, à proximité de sa périphérie.

Selon d'autres caractéristiques, le carter a une forme générale de disque. Le fluide est un liquide ou de l'air.

Le dispositif trouve une application particulièrement avantageuse pour la mise en tension d'un implant d'incontinence.

L'invention est exposée ci-après plus en détail à l'aide des figures des dessins annexés dans lesquels :
- la figure 1 est une vue en perspective avant assemblage des principaux éléments du dispositif selon l'invention ;
- la figure 2 est une vue en perspective du dispositif après assemblage des différents éléments ;
- la figure 3 est une vue en coupe longitudinale du dispositif ;

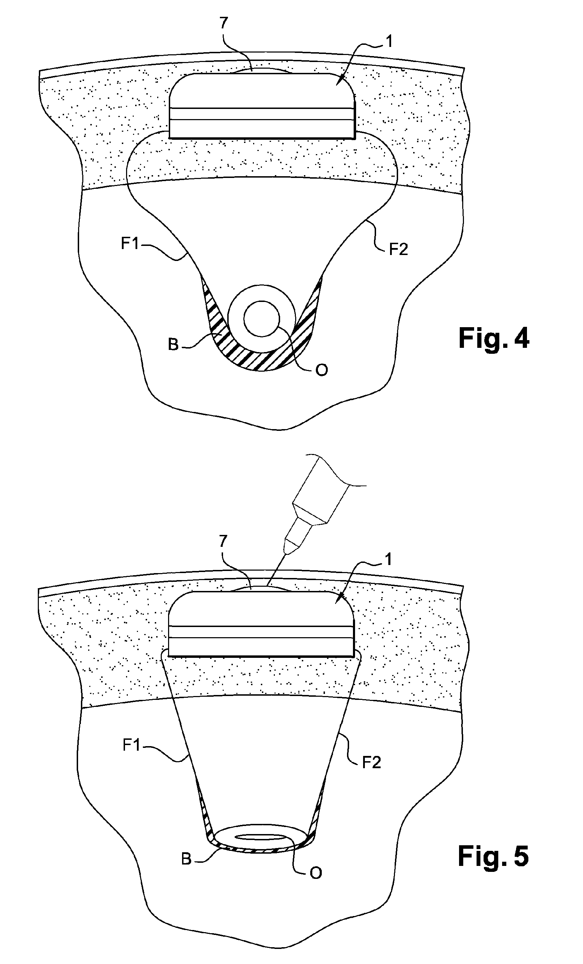

- les figures 4 et 5 sont des vues à caractère purement schématique montrant un exemple d'application nullement limitatif du dispositif selon l'invention :
   ✔ à la figure 4, les fils reliant l'implant au dispositif ne sont pas soumis à l'effort de traction;
   ✔ tandis que la figure 5, l'implant est mis sous tension suite à l'effort de traction exercé sur les fils d'accouplement.

Comme indiqué, l'invention trouve une application avantageuse dans le cas de tout type d'implant de soutènement d'un implant anatomique interne sur lequel il est nécessaire d'exercer une tension avec capacité de réglage afin d'agir sur ledit organe anatomique. C'est particulièrement le cas d'un implant d'incontinence qui se présente, par exemple, sous forme d'une bandelette (B) en matériau souple et destinée à coopérer, par exemple, avec le canal de l'urètre (O). Chacune des extrémités de la bandelette (B) ou autre implant, est reliée à des fils de traction (F1) et (F2) destinés à être accouplés au dispositif de mise en tension.

Selon l'invention, le dispositif de mise en tension comprend, logé dans un carter de protection étanche et implantable (1), un moyen d'entraînement en rotation d'un disque (2) où sont accouplés les fils de traction (F1) et (F2).

Comme il sera indiqué dans la suite de la description, le moyen d'entraînement du disque (2) est commandé par injection d'un fluide à partir d'une chambre implantable (7) que présente une partie du carter (1).

Le disque (2) est monté libre à rotation dans une embase circulaire (3) à partir de laquelle sont introduits les fils de traction (F1) et (F2). Plus particulièrement les fils (F1) et (F2) sont introduits respectivement à partir de deux trous ou orifices diamétralement opposés (3a) et (3b) pour être accouplés dans des orifices ou autres agencements (2a) et (2b) formés d'une manière diamétralement opposée à proximité de la périphérie du disque (2).

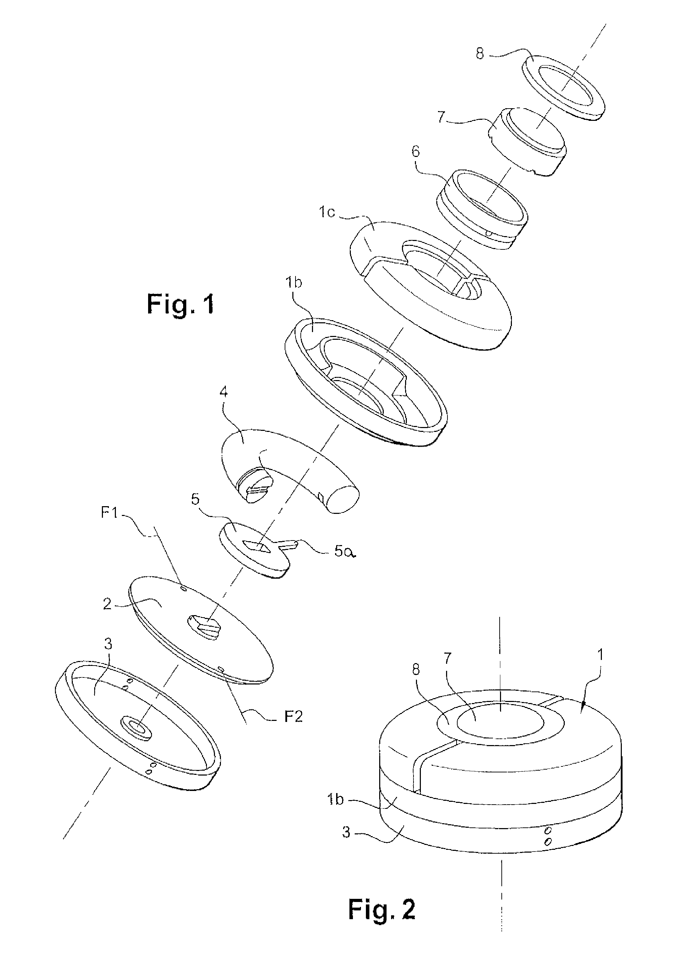

Le moyen d'entraînement du disque (2) est constitué par un piston (4) monté avec capacité de déplacement guidé et étanche dans un logement (1a) que présente le carter (1).
Comme le montre notamment la figure 1, le piston (4) a une forme générale semi-circulaire, d'une manière complémentaire à celle de son logement (1a).

Dans l'exemple de réalisation illustrée, le carter (1) est réalisé sous forme de deux demi-coquilles assemblables (1b) et (1c) délimitant le logement (1a) pour le déplacement du piston (4) et l'injection du fluide. L'une des parties (1b) ou (1c) du carter présente un agencement (1) faisant office de butée au piston (4).

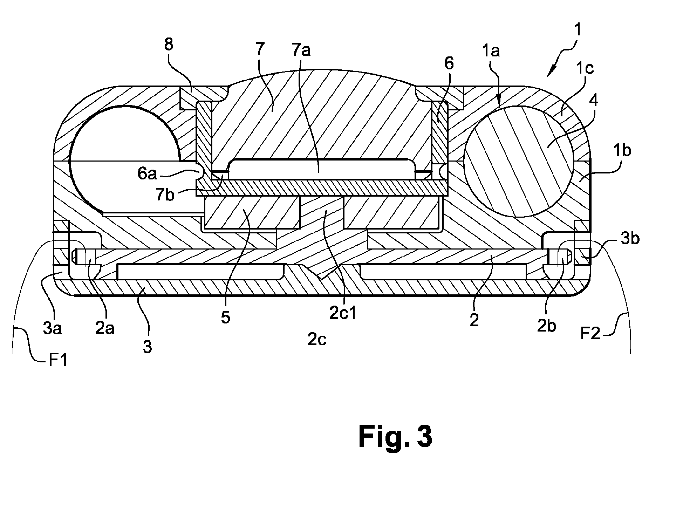

Le disque (2) présente coaxialement un doigt (5) indexé angulairement pour permettre l'entraînement en rotation du disque sous un effet de déplacement du piston (4) résultant de l'injection du fluide. Dans ce but, le doigt (5) présente un ergot (5a) débordant dans le logement (1a) du carter (1). On renvoie à la figure 3 qui montre un exemple de réalisation du montage du disque (2) par rapport au carter. Dans cette forme de réalisation, le disque (2) présente une portée circulaire de centrage (2c) sur laquelle est montée la partie inférieure (1b) du carter. Cette portée circulaire de centrage (2c) présente un bossage coaxial (2c1) de section méplate coopérant avec une fente de section complémentaire que présente le doigt (5).

Le carter (1) présente un évidement coaxial dans lequel est monté un support (6) destiné à recevoir une chambre sous-cutanée (7). Le fond de la chambre (7) présente un évidement circulaire (7a) en communication par des canaux (7b) avec le support (6) lui-même en communication par des orifices (6a) avec le logement (1a) du carter (1). Un couvercle (8) assure la liaison de la chambre (7) et de son support (6) par rapport au carter (1).

La chambre (7) est réalisée dans un matériau déformable de tout type connu et approprié, et conformé pour permettre d'une manière parfaitement connue pour un homme du métier, de planter l'aiguille d'une seringue par exemple (9), pour l'injection d'un fluide de forme liquide ou gazeuse.

Le fonctionnement du dispositif pour la mise en tension de la bandelette (B) est particulièrement simple et efficace.

On considère que ce dernier est convenablement implanté dans la partie anatomique du corps humain. L'organe anatomique considéré, le canal de l'urètre (O) par exemple, coopérant avec la bandelette (B) dont les extrémités sont reliées par les fils (F1) et (F2) au disque rotatif (2) dans les conditions indiquées précédemment.
Lorsque le fluide est injecté au travers de la chambre implantable (7) dans le logement (1a) du carter (1), le piston (4) vient en butée contre l'ergot (5a) du doigt (5) qui est déplacé sous l'effet de la poussée du fluide provoquant, d'une manière concomitante, le déplacement circulaire de l'ensemble du disque (2) et de son doigt d'entraînement (5).
Le déplacement du disque (2), auquel sont accouplé les fils (F1) et (F2), soumet lesdits fils à un effort de traction (figure 6) correspondant à la mise en tension de la bandelette (B). L'effort de traction peut facilement être réglé en fonction du débit du fluide en combinaison, par exemple, avec des témoins radio opaques que présente une partie du disque (2) afin de permettre de repérer, par radiographie, sa position angulaire pour régler par conséquent cet effort de traction desdits fils (F1) et (F2).

Pour le relâchement, il suffit de retirer, au moyen de la seringue, le fluide injecté, le piston (4) revenant en position sous l'effet de l'entraînement en sens inverse du disque (2), par le poids de l'organe anatomique considéré.

Bien évidemment, l'ensemble des principaux organes du dispositif, tel que décrit, sont réalisés en tout matériau implantable et compatible, afin d'éviter tout phénomène de rej et.

Les avantages ressortent bien de la description, en particulier on souligne et on rappelle :
- la tension ou le relâchement de l'implant s'effectue au moyen d'une seringue à travers une chambre implantable, sans incision ;
- la tension des fils s'effectue par déplacement circulaire d'un disque, d'une manière complètement symétrique, évitant tout problème de déformation ;
- la capacité d'obtenir un réglage jusqu'à 80 millimètres sans déformation;
- l'encombrement réduit du dispositif sous forme d'un ensemble compact.

## Revendications

1. Dispositif de mise en tension d'un implant en matériau souple assujetti à un ou des fil(s) de traction (F1) et (F2) et destiné à coopérer avec un organe anatomique interne, **caractérisé en ce qu'**il comprend, logé dans un carter de protection étanche et implantable (1), un moyen (4) d'entraînement en rotation d'un disque (2) où sont accouplés le ou le(s) fils de traction (F1) et (F2), ledit moyen d'entraînement (4) étant commandé par injection d'un fluide à partir d'une chambre (7) que présente ledit carter (1).

2. Dispositif selon la revendication 1, **caractérisé en ce que** le moyen d'entraînement est un piston (4) monté avec capacité de déplacement guidé et étanche dans un logement (1a) du carter (1) dans lequel est injecté le fluide.

3. Dispositif selon les revendications 1 et 2, **caractérisé en ce que** le disque (2) d'accouplement du ou des fil(s) présente une partie (5) débordant dans l'alésage (1a) du carter et apte à être sollicitée, sous un effet de déplacement du piston (4), pour provoquer, d'une manière concomitante, l'entraînement en rotation dudit disque (2).

4. Dispositif selon la revendication 2, **caractérisé en ce que** le piston (4) a une forme semi-circulaire, d'une manière complémentaire à celle de son logement.

5. Dispositif selon l'une des revendications 1 à 4, **caractérisé en ce que** la chambre implantable (7) est fixée dans un support (6) coaxial au carter (1) et en communication par au moins un orifice avec le logement du piston (4).

6. Dispositif selon la revendication 1, **caractérisé en ce que** les fils de traction (F1) et (F2) de l'implant, sont accouplés à deux orifices diamétralement opposés, formés dans l'épaisseur du disque (2), à proximité de sa périphérie.

7. Dispositif selon la revendication 1, **caractérisé en ce que** le carter (1) a une forme générale de disque.

8. Dispositif selon la revendication 1, **caractérisé en ce que** le fluide est un liquide.

9. Dispositif selon la revendication 1, **caractérisé en ce que** le fluide est de l'air.
